# EUROPEAN PATENT APPLICATION

(11) **EP 4 502 135 A1**
(43) Date of publication of application: **05.02.2025**
(21) Application number: 23780698.9
(22) Date of filing: 29.03.2023
(51) Int. Cl.: C12N 1/00

(54) **METHOD FOR PRODUCING MIXED POWDER, MIXED POWDER, POWDER, AND POWDER CULTURE MEDIUM**

(30) Priority: 30.03.2022 JP 2022056103
(71) Applicant: FUJIFILM Corporation, Tokyo 106-8620 (JP)
(72) Inventor: SHIMONO Katsuhiro, Ashigarakami-gun, Kanagawa 258-8577 (JP)
(74) Representative: Hoffmann Eitle
(86) International application number: PCT/JP2023/012814
(87) International publication number: WO 2023/190689

(57) **Abstract**

An object of the present invention is to provide a manufacturing method of a powder for obtaining a powder having excellent solubility and having a liquid property neutral (pH of 6.0 to 8.5) in a case of being dissolved in water, with respect to insoluble cystine, and to provide the powder and a powder medium containing the powder. The manufacturing method of a mixed powder of the present invention includes a dissolving step of dissolving cystine and a specific component which is at least one selected from the group consisting of tryptophan, threonine, and serine in a solvent to obtain a solution containing the cystine and the specific component, and a powdering step of drying the solution to obtain a mixed powder of the cystine and the specific component.

## Description

### BACKGROUND OF THE INVENTION

### 1. Field of the Invention

The present invention relates to a manufacturing method of a mixed powder, a mixed powder, a powder, and a powder medium.

### 2. Description of the Related Art

In the related art, in order to improve solubility of an insoluble medium, a method of freeze-drying from an aqueous solution with other components has been proposed (JP2018-521682A).

### SUMMARY OF THE INVENTION

In such a case, the present inventor has found that, in a case where an aqueous solution of cystine insoluble in water is prepared with another component (hereinafter, also referred to as "second component") and then freeze-dried with reference to JP2018-521682A, solubility of the obtained mixed powder is insufficient depending on the type of the second component. In addition, it has been found that, depending on the type of the second component, the obtained mixed powder had a basic liquid property in a case where the mixed powder is dissolved in water. In a case where the liquid property is basic, there is a problem that the liquid may be decomposed in a case where a component such as a vitamin is present in the medium.

In view of the above-described circumstances, an object of the present invention is to provide a manufacturing method of a powder for obtaining a powder having excellent solubility and having a liquid property neutral (pH of 6.0 to 8.5) in a case of being dissolved in water, with respect to insoluble cystine, and to provide the powder and a powder medium containing the powder.

As a result of intensive studies on the above-described objects, the present inventor has found that the above-described objects can be achieved by using, as a second component, at least one component selected from the group consisting of tryptophan, threonine, and serine, and has completed the present invention.

That is, the present inventors have found that the above-described objects can be achieved by the following configurations.
(1) A manufacturing method of a mixed powder, comprising:
   a dissolving step of dissolving cystine and a specific component which is at least one selected from the group consisting of tryptophan, threonine, and serine in a solvent to obtain a solution containing the cystine and the specific component; and
   a powdering step of drying the solution to obtain a mixed powder of the cystine and the specific component.
(2) The manufacturing method of a mixed powder according to (1),
   in which, in the dissolving step, heating and/or an alkali is used for the dissolution.
(3) The manufacturing method of a mixed powder according to (1) or (2),
   in which, in the dissolving step, heating is used for the dissolution.
(4) The manufacturing method of a mixed powder according to any one of (1) to (3),
   in which the powdering step is a step of drying the solution by spray drying and/or a step of drying the solution by heating and decompressing.
(5) The manufacturing method of a mixed powder according to any one of (1) to (4),
   in which, in the dissolving step, a mixed ratio of the specific component to the cystine is 1 to 10 in terms of mass ratio.
(6) A mixed powder manufactured by the manufacturing method of a mixed powder according to any one of (1) to (5).
(7) A mixed powder comprising:
   cystine; and
   a specific component which is at least one selected from the group consisting of tryptophan, threonine, and serine,
   in which a solution obtained by dissolving the mixed powder in water at 15°C to 25°C for 7 minutes has a pH of 6.0 to 8.5, and
   a cystine concentration of the obtained solution is 0.12 g/L or more.
(8) A powder comprising:
   cystine; and
   a specific component which is at least one selected from the group consisting of tryptophan, threonine, and serine,
   in which, in a case where the powder is completely dissolved in water at 15°C to 25°C such that a final concentration of the cystine is 0.14 g/L or more, the following requirement is satisfied,
   the requirement: in a case where a time when water is added with a water amount for adjusting the final concentration is set as 0 minutes, the solution is sampled after 7 minutes and filtered through a 0.2 µm filter, and the obtained liquid has a pH of 8.5 or less and a concentration of the cystine of 0.12 g/L or more.
(9) A powder medium comprising:
   the mixed powder or the powder according to any one of (6) to (8).

As shown below, according to the present invention, it is possible to provide a manufacturing method of a powder for obtaining a powder having excellent solubility and having a liquid property neutral (pH of 6.0 to 8.5) in a case of being dissolved in water, with respect to insoluble cystine, and to provide the powder and a powder medium containing the powder.

### DESCRIPTION OF THE PREFERRED EMBODIMENTS

Hereinafter, the manufacturing method of a mixed powder according to the embodiment of the present invention and the mixed powder according to the embodiment of the present invention will be described.

In the present specification, numerical ranges represented by "to" include numerical values before and after "to" as lower limit values and upper limit values.

In addition, each component may be used alone or in combination of two or more thereof. Here, in a case where two or more kinds are used in combination for each component, a content with regard to the component indicates the total content thereof, unless otherwise specified.

### [1] Manufacturing method of mixed powder

The manufacturing method of a mixed powder according to the embodiment of the present invention (hereinafter, also simply referred to as "manufacturing method according to the embodiment of the present invention") includes a dissolving step of dissolving cystine and a specific component which is at least one selected from the group consisting of tryptophan, threonine, and serine in a solvent to obtain a solution containing the cystine and the specific component, and a powdering step of drying the solution to obtain a mixed powder of the cystine and the specific component.

It is considered that the above-described object can be achieved by adopting such a configuration of the manufacturing method according to the embodiment of the present invention. The reason is not clear, but it is considered as follows.

As one factor of insolubility of cystine, aggregation of hydrophobic portions between molecules of cystine is considerable. On the other hand, in the present invention, since cystine and the specific component are dissolved in a solvent and then dried, it is considered that the specific component enters between the molecules of cystine, and the formation of the hydrophobic portion by the aggregation of the molecules of cystine is suppressed, so that the solvent (particularly, a hydrophilic solvent) easily permeates. Furthermore, the specific component exhibits excellent solubility. Therefore, according to the present invention, it is considered that a powder in which the cystine and highly soluble specific component are mixed with each other at a molecular level is obtained. As a result, it is considered that the mixed powder obtained by the manufacturing method according to the embodiment of the present invention exhibits excellent solubility. This is presumed from the fact that the solubility is not improved even in a case where the cystine and specific component are simply mixed.

Hereinafter, each step of the manufacturing method according to the embodiment of the present invention will be described.

### [Dissolving step]

The dissolving step is a step of dissolving cystine and the specific component in a solvent to obtain a solution containing cystine and the specific component.

### [Cystine]

A mixing amount of the cystine in the solvent is not particularly limited, but from the reason that the effect of the present invention is more excellent, in a case of using heating for the dissolution, it is preferably 0.01 to 100 g/L, more preferably 0.05 to 10 g/L, and still more preferably 0.1 to 2 g/L; and in a case of using an alkali for the dissolution, it is preferably 0.1 to 1,000 g/L, more preferably 1 to 200 g/L, and still more preferably 10 to 100 g/L.

### [Specific component]

The specific component is at least one selected from the group consisting of tryptophan, threonine, and serine. From the reason that the effect of the present invention is more excellent, the specific component is preferably threonine or serine, and more preferably threonine.

A mixing amount of the specific component in the solvent is not particularly limited, but from the reason that the effect of the present invention is more excellent, in a case of using heating for the dissolution, it is preferably 0.01 to 1,000 g/L, more preferably 0.1 to 100 g/L, and still more preferably 1 to 5 g/L; and in a case of using an alkali for the dissolution, it is preferably 1 to 3,000 g/L, more preferably 10 to 2,000 g/L, and still more preferably 100 to 1,000 g/L.

### [Mixed ratio]

From the reason that the effect of the present invention is more excellent, a mixed ratio of the specific component to the cystine (mixing amount of specific component/mixing amount of c) is preferably 0.1 to 100, more preferably 0.5 to 80, still more preferably 1 to 60, particularly preferably 2 to 40, and most preferably 5 to 20 in terms of mass ratio. Hereinafter, the mixed ratio of the second component to the cystine (mixing amount of second component/mixing amount of cystine) (mass ratio) will be simply referred to as "mixed ratio".

### [Solvent]

The solvent used in the dissolving step is not particularly limited, but from the reason that the effect of the present invention is more excellent, water or a hydrophilic solvent is preferable, and water is more preferable. In addition, the mixture may be liquefied during heating, and may be solid at room temperature.

The hydrophilic solvent is not particularly limited as long as it is a solvent having hydrophilicity, and specific examples thereof include ether group-containing compounds such as tetrahydrofuran, dimethoxyethane, diethylene glycol dimethyl ether, and polyethylene glycol dialkyl ether; lactone group-containing compounds such as γ-butyrolactone and γ-valerolactone; amide or lactam group-containing compounds such as caprolactam, N-methylcaprolactam, N,N-dimethylacetamide, N-methylacetamide, N,N-dimethylformamide (DMF), N-methylformamide, N-methylformanilide, N-methylpyrrolidone (NMP), N-octylpyrrolidone, and pyrrolidone; sulfone and sulfoxide such as sulfolane (tetramethylene sulfone) and dimethyl sulfoxide (DMSO); sugar or sugar derivatives such as sucrose, glucose, fructose, and lactose; sugar alcohols such as sorbitol and mannitol; furan derivatives such as 2-furancarboxylic acid and 3-furancarboxylic acid; alcohols such as methanol, ethylene glycol, glycerol, diethylene glycol, and triethylene glycol; and phenol compounds such as dimethoxyphenol, m-cresol, o-cresol, p-cresol, phenol, and naphthol. These hydrophilic solvents may be used alone or in combination of two or more kinds thereof.

### [Dissolving method]

A method for dissolving the cystine and the specific component in the solvent is not particularly limited, but from the reason that the effect of the present invention is more excellent, it is preferable to use heating and/or an alkali, and it is more preferable to use heating. The expression "use heating" means heating the solvent, and the expression "use an alkali" means adding an alkali (sodium hydroxide or the like) to the solvent.

A temperature in a case of using heating for the dissolution is not particularly limited, but from the reason that the effect of the present invention is more excellent, it is preferably 40°C to 300°C, preferably 40°C to 200°C, and more preferably 60°C to 90°C. The solvent may be in a subcritical or supercritical state with pressurization at a temperature equal to or higher than a boiling point, during the dissolution.

In a case where an alkali is used for the dissolution, from the reason that the effect of the present invention is more excellent, it is preferable that a pH of the solution is 12 or more. In addition, in a case where an alkali is used for the dissolution, from the reason that the effect of the present invention is more excellent, it is preferable to adjust the pH to be neutral after the dissolution using an acid (hydrochloric acid or the like) (neutralizing step). The term "neutral" in the present specification means a pH of 6.0 to 8.5.

In a case where an alkali is used for the dissolution and then the pH of the solution is adjusted to be neutral using an acid, the obtained solution contains a salt. In a case where the solution contains a salt, it is considered that the suppression of the association between the molecules of cystine due to the specific component is slightly reduced. In addition, in a case where the solution contains a salt, the obtained mixed powder also contains the salt. In a case where the mixed powder contains the salt, there is a concern that osmotic pressure may increase in a case of being dissolved in water. For such a reason, it is preferable to use heating rather than using an alkali for the dissolution.

### [Powdering step]

The powdering step is a step of drying the solution obtained by the above-described dissolving step to obtain a mixed powder of cystine and the specific component.

### [Drying method]

A method for drying the solution is not particularly limited, and specific examples thereof include spray drying, drying (for example, drying using an evaporator) by heating (for example, at 40°C to 80°C) and decompression (for example, 100 mmHg or less), and freeze drying.

As the method for drying the solution, from the viewpoint that the obtained mixed powder has high uniformity and thus has excellent solubility, and from the viewpoint that the drying time is short, a drying method other than the freeze drying is preferable, spray drying and/or drying by heating and decompression is more preferable, and spray drying is still more preferable.

### [Mixed powder]

The mixed powder obtained by the manufacturing method according to the embodiment of the present invention is particularly useful as a powder for a culture medium.

### [2] Mixed powder

The mixed powder according to the embodiment of the present invention is a mixed powder of cystine and a specific component which is at least one selected from the group consisting of tryptophan, threonine, and serine, in which an aqueous solution obtained by dissolving the mixed powder in water at 15°C to 30°C (preferably, 15°C to 25°C) for 7 minutes has a pH of 6.0 to 8.5, and a cystine concentration of the obtained solution is 0.12 g/L or more.

The cystine concentration is preferably 0.12 g/L or more and 0.5 g/L or less, more preferably 0.13 g/L or more and 0.4 g/L or less, and most preferably 0.15 g/L or more and 0.3 g/L or less.

It is preferable that the above-described mixed powder is a mixed powder obtained by the manufacturing method according to the embodiment of the present invention described above.

### Examples

Hereinafter, the present invention will be described in more detail with reference to Examples, but the present invention is not limited thereto.

### [Production of powder]

Each powder was produced as follows.

### [Example 1]

### <Dissolving step>

500 mL of distilled water, 80 mg of L-cystine (0.33 mmol, manufactured by FUJIFILM Wako Pure Chemical Corporation), and 240 mg of L-tryptophan (1.17 mmol, manufactured by FUJIFILM Wako Pure Chemical Corporation) were put into a 1000 mL three-neck flask equipped with a stirring blade, the mixture was heated at 80°C for 2 hours with stirring at 300 rpm (rotation/min), and it was confirmed that the L-cystine and the L-tryptophan were completely dissolved. In this way, a supersaturated solution was obtained. The obtained supersaturated solution is also referred to as A1. A1 was stable for several hours even after being returned to room temperature.

### <Powdering step>

The supersaturated solution (A1) obtained in the dissolving step was put into a 1000 mL flask. The mixture was heated (at 50°C to 60°C) and depressurized (50 mmHg or less) using an evaporator to be dried. In this way, a powder was obtained. The obtained powder is also referred to as CT1.

In a case where the composition of CT1 was confirmed by a ¹H-nuclear magnetic resonance (NMR) method, the powder was a mixed powder of cystine:tryptophan = 1:3.5 (molar ratio).

### [Examples 2 and 3]

A mixed powder of cystine and tryptophan was obtained by the same procedure as in Example 1, except that the mixing amount of the L-tryptophan in the dissolving step was changed as shown in Table 1.

### [Example 4]

A mixed powder of cystine and tryptophan was obtained by the same procedure as in Example 1, except that, in the powdering step, the supersaturated solution obtained in the dissolving step was spray-dried using a mini-spray dryer B-290 (manufactured by BUCHI Corporation). In a case where the supersaturated solution was insufficient in a case of obtaining the powder, additional supersaturated solution was prepared and used.

### [Examples 5 and 6]

A mixed powder of cystine and tryptophan was obtained by the same procedure as in Example 4, except that the mixing amount of the L-tryptophan in the dissolving step was changed as shown in Table 1.

### [Example 7]

A mixed powder of cystine and tryptophan was obtained by the same procedure as in Example 1, except that, in the powdering step, the supersaturated solution obtained in the dissolving step was freeze-dried using a freeze dryer FDU-12AS (manufactured by AS ONE Corporation) for 1 week to obtain a powder.

### [Example 8]

A mixed powder of cystine and tryptophan was obtained by the same procedure as in Example 7, except that the mixing amount of the L-tryptophan in the dissolving step was changed as shown in Table 1.

### [Example 9]

### <Dissolving step>

30 mL of distilled water, 2.0 g of L-cystine (8.32 mmol, manufactured by FUJIFILM Wako Pure Chemical Corporation), and 6.0 g (29.3 mmol) of L-tryptophan were put into a 100 mL beaker, 4.23 g of sodium hydroxide (manufactured by FUJIFILM Wako Pure Chemical Corporation) was further added thereto, and the mixture was once completely dissolved to have a pH of 12 or more. Thereafter, an HCl aqueous solution (manufactured by FUJIFILM Wako Pure Chemical Corporation) of 5 N (normality) was added thereto to adjust the pH to be 7 to 8. In this way, a slurry solution was obtained. The obtained slurry solution is also referred to as NA1.

### <Powdering step>

The slurry solution (NA1) obtained in the dissolving step was put into a 1000 mL flask. The mixture was heated (at 50°C to 60°C) and depressurized (50 mmHg or less) using an evaporator to be dried. In this way, a powder was obtained. The obtained powder is also referred to as CT9. In a case where the composition of CT9 was confirmed by a ¹H-NMR method, the powder was a mixed powder of cystine:tryptophan = 1:3.5 (molar ratio).

### [Examples 10 and 11]

A mixed powder of cystine and tryptophan was obtained by the same procedure as in Example 9, except that the mixing amount of the L-tryptophan in the dissolving step was changed as shown in Table 1.

### [Example 12]

A mixed powder of cystine and tryptophan was obtained by the same procedure as in Example 9, except that, in the powdering step, the slurry solution obtained in the dissolving step was freeze-dried using a freeze dryer FDU-12AS (manufactured by AS ONE Corporation) for 1 week to obtain a powder.

### [Examples 13 and 14]

A mixed powder of cystine and tryptophan was obtained by the same procedure as in Example 12, except that the mixing amount of the L-tryptophan in the dissolving step was changed as shown in Table 1.

### [Examples 15 to 28]

Mixed powders of cystine and serine were obtained by the same procedure as in Examples 1 to 14, except that, in the dissolving step, L-serine was used instead of the L-tryptophan.

### [Examples 29 to 42]

Mixed powders of cystine and threonine were obtained by the same procedure as in Examples 1 to 14, except that, in the dissolving step, L-threonine was used instead of the L-tryptophan.

### [Comparative Example 1]

### <Dissolving step>

500 mL of distilled water and 80 mg of L-cystine (0.33 mmol, manufactured by FUJIFILM Wako Pure Chemical Corporation) were put into a 1000 mL three-neck flask equipped with a stirring blade, the mixture was heated at 80°C for 2 hours with stirring at 300 rpm, and it was confirmed that the L-cystine was completely dissolved. In this way, a supersaturated solution was obtained. The obtained supersaturated solution is also referred to as D1.

### <Powdering step>

The supersaturated solution (D1) obtained in the dissolving step was put into a 1000 mL flask. The mixture was heated (at 70°C) and depressurized (50 mmHg or less) using an evaporator to be dried. In this way, a powder was obtained. The obtained powder is also referred to as CH1.

### [Comparative Example 2]

L-cystine (manufactured by FUJIFILM Wako Pure Chemical Corporation) was used as a powder of Comparative Example 2.

### [Comparative Example 3]

A powder was obtained by the same procedure as in Example 1, except that, in the dissolving step, L-phenylalanine was used instead of the L-tryptophan. The obtained powder is also referred to as CH2.

In a case where the composition of CH2 was confirmed by a ¹H-NMR method, the powder was a mixed powder of cystine:phenylalanine = 1:6.9 (molar ratio).

### [Comparative Example 4]

A powder was obtained by the same procedure as in Example 1, except that, in the dissolving step, L-arginine was used instead of the L-tryptophan. The obtained powder is also referred to as CH3.

In a case where the composition of CH3 was confirmed by a ¹H-NMR method, the powder was a mixed powder of cystine:arginine = 1:6.9 (molar ratio).

### [Evaluation]

The following evaluations were performed on each of the obtained powders.

### [Solubility]

30 mL of distilled water was put into a 100 mL beaker, and the powder was added thereto with stirring using a stirrer chip (25°C). The addition amount of the powder was as shown in the column of "Addition amount" in Tables 1 to 3. Solubility was visually evaluated according to the following evaluation standard with the addition time being 0 minutes.

The results are shown in Tables 1 to 3. In practice, AA, A, B, C, or D is preferable; AA, A, B, or C is more preferable; AA, A, or B is still more preferable; AA or A is particularly preferable; and AA is most preferable.

The above-described addition amount was calculated from the mixed ratio such that the amount of cystine in the solution was 0.17 g/L.
· AA: completely dissolved within 30 seconds
· A: completely dissolved in more than 30 seconds and 5 minutes or less
· B: completely dissolved in more than 5 minutes and 10 minutes or less
· C: completely dissolved in more than 10 minutes and 15 minutes or less.
· D: completely dissolved in 15 minutes or more and 1 hour or less.
· E: turbidity and sediment were observed even after 1 hour.

### [Liquid property]

A pH of the solution obtained in the evaluation of solubility was evaluated. Liquid property was evaluated according to the following evaluation standard. The results are shown in Tables 1 to 3. In practice, the pH is preferably neutral.
· Acidic: pH of less than 6.0
· Neutral: pH of 6.0 to 8.5
· Basic: pH of more than 8.5

### [Overall evaluation]

Based on the evaluation results of solubility and liquid property, the overall evaluation was performed according to the following evaluation standard. The results are shown in Tables 1 to 3.
· AA: completely dissolved within 30 seconds, and the liquid property was neutral.
· A: completely dissolved within 5 minutes, and the liquid property was neutral.
· B: completely dissolved within 10 minutes, and the liquid property was neutral.
· C: completely dissolved within 15 minutes, and the liquid property was neutral.
· D: completely dissolved within 1 hour, and the liquid property was neutral.
· E: the solution was still turbid even after 1 hour, and/or the pH was other than neutral.

**[Table 1]**

| | Dissolving step | | | | | | | | Powdering step | | | | Evaluation | | | |
|---|---|---|---|---|---|---|---|---|---|---|---|---|---|---|---|---|
| | Distilled water [mL] | L-cystine [mg] | Second component | | Mixed ratio (mass ratio) | Dissolving method | Solvent | | Drying method | Powder | | | Addition amount [mg] (cystine: 0.17g/L) | Solubility | Liquid property | Overall evaluation |
| | | | Type | Mixing amount [mg] | | | Abbreviation | Liquid state | | Abbreviation | Compositional ratio (molar ratio) | Salt content (%) | | | | |
| Example 1 | 500 | 80 | L-tryptophan | 240 | 3 | Heating | A1 | Uniform | Evaporator | CT1 | 3.5 | 0 | 20.4 | A | Neutral | A |
| Example 2 | 500 | 80 | L-tryptophan | 80 | 1 | Heating | A2 | Uniform | Evaporator | CT2 | 1.2 | 0 | 102 | B | Neutral | B |
| Example 3 | 500 | 80 | L-tryptophan | 800 | 10 | Heating | A3 | Uniform | Evaporator | CT3 | 11.8 | 0 | 56.1 | A | Neutral | A |
| Example 4 | 500 | 80 | L-tryptophan | 240 | 3 | Heating | A1 | Uniform | Spray drying | CT4 | 3.5 | 0 | 20.4 | AA | Neutral | AA |
| Example 5 | 500 | 80 | L-tryptophan | 61 | 0.76 | Heating | A4 | Uniform | Spray drying | CT5 | 0.89 | 0 | 9.0 | B | Neutral | B |
| Example 6 | 500 | 80 | L-tryptophan | 800 | 10 | Heating | A3 | Uniform | Spray drying | CT6 | 11.8 | 0 | 56.1 | AA | Neutral | AA |
| Example 7 | 500 | 80 | L-tryptophan | 240 | 3 | Heating | A1 | Uniform | Freeze drying | CT7 | 3.5 | 0 | 20.4 | B | Neutral | B |
| Example 8 | 500 | 80 | L-tryptophan | 80 | 1 | Heating | A2 | Uniform | Freeze drying | CT8 | 1.2 | 0 | 102 | B | Neutral | B |
| Example 9 | 30 | 2000 | L-tryptophan | 6000 | 3 | Alkali | NA1 | Slurry | Evaporator | CT9 | 3.5 | 36 | 27.7 | B | Neutral | B |
| Example 10 | 30 | 2000 | L-tryptophan | 20000 | 10 | Alkali | NA2 | Slurry | Evaporator | CT10 | 11.8 | 45 | 81.3 | B | Neutral | B |
| Example 11 | 30 | 2000 | L-tryptophan | 2000 | 1 | Alkali | NA3 | Slurry | Evaporator | CT11 | 1.2 | 33 | 13.6 | C | Neutral | C |
| Example 12 | 30 | 2000 | L-tryptophan | 6000 | 3 | Alkali | NA1 | Slurry | Freeze drying | CT12 | 3.5 | 37 | 27.9 | B | Neutral | B |
| Example 13 | 30 | 2000 | L-tryptophan | 20000 | 10 | Alkali | NA2 | Slurry | Freeze drying | CT13 | 11.8 | 45 | 81.3 | B | Neutral | B |
| Example 14 | 30 | 2000 | L-tryptophan | 2000 | 1 | Alkali | NA3 | Slurry | Freeze drying | CT14 | 1.2 | 33 | 13.6 | C | Neutral | C |

**[Table 2]**

| | Dissolving step | | | | | | | | Powdering step | | | | Evaluation | | | |
|---|---|---|---|---|---|---|---|---|---|---|---|---|---|---|---|---|
| | Distilled water [mL] | L-cystine [mg] | Second component | | Mixed ratio (mass ratio) | Dissolving method | Solvent | | Drying method | Powder | | | Addition amount [mg] (cystine: 0.17g/L) | Solubility | Liquid property | Overall evaluation |
| | | | Type | Mixing amount [mg] | | | Abbreviation | Liquid state | | Abbreviation | Compositional ratio (molar ratio) | Salt content (%) | | | | |
| Example 15 | 500 | 80 | L-serine | 240 | 3 | Heating | B1 | Uniform | Evaporator | CS1 | 6.9 | 0 | 20.4 | A | Neutral | A |
| Example 16 | 500 | 80 | L-serine | 80 | 1 | Heating | B2 | Uniform | Evaporator | CS2 | 2.3 | 0 | 102 | B | Neutral | B |
| Example 17 | 500 | 80 | L-serine | 800 | 10 | Heating | B3 | Uniform | Evaporator | CS3 | 229 | 0 | 56.1 | A | Neutral | A |
| Example 18 | 500 | 80 | L-serine | 240 | 3 | Heating | B1 | Uniform | Spray drying | CS4 | 6.9 | 0 | 20.4 | AA | Neutral | AA |
| Example 19 | 500 | 80 | L-serine | 61 | 0.76 | Heating | B4 | Uniform | Spray drying | CS5 | 1.7 | 0 | 9.0 | C | Neutral | C |
| Example 20 | 500 | 80 | L-serine | 800 | 10 | Heating | B3 | Uniform | Spray drying | CS6 | 228 | 0 | 56.1 | AA | Neutral | AA |
| Example 21 | 500 | 80 | L-serine | 240 | 3 | Heating | B1 | Uniform | Freeze drying | CS7 | 6.9 | 0 | 20.4 | B | Neutral | B |
| Example 22 | 500 | 80 | L-serine | 80 | 1 | Heating | B2 | Uniform | Freeze drying | CS8 | 2.3 | 0 | 102 | B | Neutral | B |
| Example 23 | 30 | 2000 | L-serine | 6000 | 3 | Alkali | NB1 | Slurry | Evaporator | CS9 | 6.9 | 25 | 25.5 | B | Neutral | B |
| Example 24 | 30 | 2000 | L-serine | 20000 | 10 | Alkali | NB2 | Slurry | Evaporator | CS10 | 228 | 35 | 75.7 | B | Neutral | B |
| Example 25 | 30 | 2000 | L-serine | 2000 | 1 | Alkali | NB3 | Slurry | Evaporator | CS11 | 2.3 | 31 | 13.4 | C | Neutral | C |
| Example 26 | 30 | 2000 | L-serine | 6000 | 3 | Alkali | NB1 | Slurry | Freeze drying | CS12 | 6.9 | 27 | 25.9 | B | Neutral | B |
| Example 27 | 30 | 2000 | L-serine | 20000 | 10 | Alkali | NB2 | Slurry | Freeze drying | CS13 | 228 | 36 | 76.3 | B | Neutral | B |
| Example 28 | 30 | 2000 | L-serine | 2000 | 1 | Alkali | NB3 | Slurry | Freeze drying | CS14 | 2.3 | 30 | 13.3 | C | Neutral | C |

**[Table 3]**

| | Dissolving step | | | | | | | | Powdering step | | | | Evaluation | | | |
|---|---|---|---|---|---|---|---|---|---|---|---|---|---|---|---|---|
| | Distilled water [mL] | L-cystine [mg] | Second component | | Mixed ratio (mass ratio) | Dissolving method | Solvent | | Drying method | Powder | | | Addition amount [mg] (cystine: 0.17g/L) | Solubility | Liquid property | Overall evaluation |
| | | | Type | Mixing amount [mg] | | | Abbreviation | Liquid state | | Abbreviation | Compositional ratio (molar ratio) | Salt content (%) | | | | |
| Example 29 | 500 | 80 | L-threonine | 240 | 3 | Heating | C1 | Uniform | Evaporator | CThr1 | 6.1 | 0 | 20.4 | A | Neutral | A |
| Example 30 | 500 | 80 | L-threonine | 80 | 1 | Heating | C2 | Uniform | Evaporator | CThr2 | 2.0 | 0 | 10.2 | B | Neutral | B |
| Example 31 | 500 | 80 | L-threonine | 800 | 10 | Heating | C3 | Uniform | Evaporator | CThr3 | 20.1 | 0 | 56.1 | A | Neutral | A |
| Example 32 | 500 | 80 | L-threonine | 240 | 3 | Heating | C1 | Uniform | Spray drying | CThr4 | 6.9 | 0 | 20.4 | AA | Neutral | AA |
| Example 33 | 500 | 80 | L-threonine | 61 | 0.76 | Heating | C4 | Uniform | Spray drying | CThr5 | 1.5 | 0 | 9.0 | C | Neutral | C |
| Example 34 | 500 | 80 | L-threonine | 800 | 10 | Heating | C3 | Uniform | Spray drying | CThr6 | 20.1 | 0 | 56.1 | AA | Neutral | AA |
| Example 35 | 500 | 80 | L-threonine | 240 | 3 | Heating | C1 | Uniform | Freeze drying | CThr7 | 6.9 | 0 | 20.4 | B | Neutral | B |
| Example 36 | 500 | 80 | L-threonine | 80 | 1 | Heating | C2 | Uniform | Freeze drying | CThr8 | 2.3 | 0 | 102 | B | Neutral | B |
| Example 37 | 30 | 2000 | L-threonine | 6000 | 3 | Alkali | NC1 | Slurry | Evaporator | CThr9 | 6.1 | 32 | 26.9 | B | Neutral | B |
| Example 38 | 30 | 2000 | L-threonine | 20000 | 10 | Alkali | NC2 | Slurry | Evaporator | CThr10 | 20.1 | 40 | 78.5 | B | Neutral | B |
| Example 39 | 30 | 2000 | L-threonine | 2000 | 1 | Alkali | NC3 | Slurry | Evaporator | CThr11 | 2.3 | 30 | 13.3 | C | Neutral | C |
| Example 40 | 30 | 2000 | L-threonine | 6000 | 3 | Alkali | NC1 | Slurry | Freeze drying | CThr12 | 6.1 | 31 | 26.7 | B | Neutral | B |
| Example 41 | 30 | 2000 | L-threonine | 20000 | 10 | Alkali | NC2 | Slurry | Freeze drying | CThr13 | 20.1 | 41 | 79.1 | B | Neutral | B |
| Example 42 | 30 | 2000 | L-threonine | 2000 | 1 | Alkali | NC3 | Slurry | Freeze drying | CThr14 | 2.0 | 31 | 13.4 | C | Neutral | C |
| Comparative Example 1 | 500 | 80 | - | - | - | Heating | D1 | Uniform | Evaporator | CH1 | - | 0.0 | 5.1 | E | Neutral | E |
| Comparative Example 2 | - | - | - | - | - | Heating | - | - | - | L-cystine | - | 0.0 | 5.1 | E | Neutral | E |
| Comparative Example 3 | 500 | 80 | L-phenylalanine | 240 | 3 | Heating | D2 | Uniform | Evaporator | CH2 | 6.9 | 0.0 | 20.4 | E | Neutral | E |
| Comparative Example 4 | 500 | 80 | L-arginine | 240 | 3 | Heating | D3 | Uniform | Evaporator | CH3 | 6.9 | 0.0 | 20.4 | B | Basic | E |

In Tables 1 to 3, "Compositional ratio (molar ratio)" of the powder indicates the compositional ratio (molar ratio) of the second component to cystine. The compositional ratio was confirmed by a ¹H-NMR method.

In addition, in Tables 1 to 3, "Salt content" of the powder indicates an amount (% by mass) of salt (NaCl) in a case where the total of cystine and the second component was set to 100% by mass.

### [Summary of table]

As shown in Tables 1 to 3, the mixed powders obtained by the manufacturing method according to the embodiment of the present invention (Examples 1 to 42) exhibited excellent solubility as compared with the powder obtained by dissolving only cystine in the solvent and drying the solution (Comparative Example 1), the commercially available cystine (Comparative Example 2), and the mixed powder obtained by using phenylalanine instead of the specific component (Comparative Example 3). In Comparative Examples 1 and 2, the powder was not completely dissolved even after 4 hours. In Comparative Example 3, the powder was hardly dissolved.

As shown in Table 3, the mixed powder obtained by using arginine instead of the specific component (Comparative Example 4) had a basic liquid property in a case of being dissolved in water. In addition, from the comparison between Example 1, Example 15, Example 29, and Comparative Examples 3 and 4 (comparison between aspects in which only the type of the second component was different), the mixed powder obtained by using, as the second component, the specific component (Examples 1, 15, and 29) exhibited more excellent solubility than the mixed powder obtained by using, as the second component, a component other than the specific component (Comparative Examples 3 and 4).

### [Examples 1 to 14 (specific component: tryptophan)]

From the comparison between Examples 1 to 3 and Examples 9 to 11 (comparison between aspects in which the drying method was using an evaporator), it was found that the mixed powders obtained in Examples 1 to 3, using heating for the dissolution, exhibited more excellent solubility.

From the comparison of Examples 1 to 3 (comparison between aspects in which only the mixed ratio was different), the mixed powders obtained in Examples 1 and 3, in which the mixed ratio was 2 or more, exhibited more excellent solubility. Similarly, from the comparison of Examples 4 to 6 (comparison between aspects in which only the mixed ratio was different), the mixed powders obtained in Examples 4 and 6, in which the mixed ratio was 2 or more, exhibited more excellent solubility. Similarly, from the comparison of Examples 9 to 11 (comparison between aspects in which only the mixed ratio was different), Examples 9 and 10 in which the mixed ratio was 2 or more exhibited more excellent solubility. Similarly, from the comparison of Examples 12 to 14 (comparison between aspects in which only the mixed ratio was different), Examples 12 and 13 in which the mixed ratio was 2 or more exhibited more excellent solubility.

From the comparison between Example 1, Example 4, and Example 7 (comparison between aspects in which only the drying method was different), the mixed powder obtained in Example 1, using an evaporator (heating and decompression) for the drying, and the mixed powder obtained in Example 4, using spray drying for the drying, exhibited more excellent solubility. Among these, Example 4 in which spray drying was used for the drying exhibited further more excellent solubility. Similarly, from the comparison between Example 3 and Example 6 (comparison between aspects in which only the drying method was different), Example 6 using spray drying for the drying exhibited more excellent solubility.

### [Examples 15 to 28 (specific component: serine)]

From the comparison between Examples 15 to 17 and Examples 23 to 25 (comparison between aspects in which the drying method was using an evaporator), it was found that the mixed powders obtained in Examples 15 to 17, using heating for the dissolution, exhibited more excellent solubility.

From the comparison of Examples 15 to 17 (comparison between aspects in which only the mixed ratio was different), the mixed powders obtained in Examples 15 and 17, in which the mixed ratio was 2 or more, exhibited more excellent solubility. Similarly, from the comparison of Examples 18 to 20 (comparison between aspects in which only the mixed ratio was different), the mixed powders obtained in Examples 18 and 20, in which the mixed ratio was 2 or more, exhibited more excellent solubility. Similarly, from the comparison of Examples 23 to 25 (comparison between aspects in which only the mixed ratio was different), Examples 23 and 24 in which the mixed ratio was 2 or more exhibited more excellent solubility. Similarly, from the comparison of Examples 26 to 28 (comparison between aspects in which only the mixed ratio was different), Examples 26 and 27 in which the mixed ratio was 2 or more exhibited more excellent solubility.

From the comparison between Example 15, Example 18, and Example 21 (comparison between aspects in which only the drying method was different), the mixed powder obtained in Example 15, using an evaporator (heating and decompression) for the drying, and the mixed powder obtained in Example 18, using spray drying for the drying, exhibited more excellent solubility. Among these, Example 18 in which spray drying was used for the drying exhibited further more excellent solubility. Similarly, from the comparison between Example 17 and Example 20 (comparison between aspects in which only the drying method was different), Example 20 using spray drying for the drying exhibited more excellent solubility.

### [Examples 29 to 42 (specific component: threonine)]

From the comparison between Examples 29 to 31 and Examples 37 to 39 (comparison between aspects in which the drying method was using an evaporator), it was found that the mixed powders obtained in Examples 29 to 31, using heating for the dissolution, exhibited more excellent solubility.

From the comparison of Examples 29 to 31 (comparison between aspects in which only the mixed ratio was different), the mixed powders obtained in Examples 29 and 31, in which the mixed ratio was 2 or more, exhibited more excellent solubility. Similarly, from the comparison of Examples 32 to 34 (comparison between aspects in which only the mixed ratio was different), the mixed powders obtained in Examples 32 and 34, in which the mixed ratio was 2 or more, exhibited more excellent solubility. Similarly, from the comparison of Examples 37 to 39 (comparison between aspects in which only the mixed ratio was different), Examples 37 and 38 in which the mixed ratio was 2 or more exhibited more excellent solubility. Similarly, from the comparison of Examples 40 to 42 (comparison between aspects in which only the mixed ratio was different), Examples 40 and 41 in which the mixed ratio was 2 or more exhibited more excellent solubility.

From the comparison between Example 29, Example 32, and Example 35 (comparison between aspects in which only the drying method was different), the mixed powder obtained in Example 29, using an evaporator (heating and decompression) for the drying, and the mixed powder obtained in Example 32, using spray drying for the drying, exhibited more excellent solubility. Among these, Example 32 in which spray drying was used for the drying exhibited further more excellent solubility. Similarly, from the comparison between Example 31 and Example 34 (comparison between aspects in which only the drying method was different), Example 34 using spray drying for the drying exhibited more excellent solubility.

Solubility in a case where the mixed powder obtained by the manufacturing method according to the embodiment of the present invention was applied to an E-MEM medium (Eagle's minimal essential medium) was evaluated as follows. Since the molecular mixed powder according to the embodiment of the present invention, which consists of cystine and the specific component which is at least one selected from the group consisting of tryptophan, threonine, and serine, has an extremely high dissolution rate of cystine, it can be said that it is possible to detect whether or not the culture medium containing the molecular mixed powder of cystine and the specific component according to the embodiment of the present invention is used in the following method.

As a culture medium, an E-MEM medium B in which cystine, tryptophan, threonine, and serine were removed was mixed with the mixed powder in a uniform mortar as shown in Table 4, thereby obtaining an E-MEM medium B-mixed powder. The E-MEM medium B could be dissolved in water at a concentration of 12.0 g/L to obtain a liquid medium.

**[Table 4]**

| | |
|---|---|
| | |

| Component | mg(/L) |
|---|---|
| Calcium chloride dihydrate | 264.9 |
| Potassium chloride | 400.0 |
| Magnesium sulfate heptahydrate | 200.7 |
| Sodium chloride | 6800.0 |
| Sodium dihydrogen phosphate monohydrate | 2530.2 |
| L-arginine hydrochloride | 126.0 |
| L-cystine | 0.0 |
| L-glutamine | 292.0 |
| L-histidine hydrochloride monohydrate | 42.0 |
| L-isoleucine | 52.0 |
| L-lysine hydrochloride | 72.5 |
| L-methionine | 15.0 |
| L-phenylalanine | 32.0 |
| L-threonine | 48.0 |
| L-tryptophan | 10.0 |
| L-tyrosine | 36.0 |
| L-valine | 46.0 |
| Calcium pantothenate | 1.0 |
| Choline chloride | 1.0 |
| Folic acid | 1.0 |
| i-inositol | 2.0 |
| Nicotinamide | 1.0 |
| Pyridoxal hydrochloride | 1.0 |
| Riboflavin | 0.1 |
| Thiamine hydrochloride | 1.0 |
| Glucose | 1000.0 |
| Phenol Red | 10.0 |
| Total [g/(L)] | 12.0 |

Using the E-MEM medium B prepared in this way, as shown in Table 5, a medium B1 in which 0.15 g/L equivalent of cystine/threonine = 1/4 (mass ratio) molecular mixed powder of the present invention was added as cystine to the EMEM medium B was prepared as Example 43; and a medium B2 in which the same amounts of cystine and threonine as used in Example 43 were added to the EMEM medium B instead of the molecular mixed powder was prepared as Comparative Example 5. The same preparation was performed for serine and tryptophan, thereby obtaining B3, B4, B5, and B6, respectively.

Under a room temperature of 22°C, each of the culture medium B1 and the culture medium B2 were put into a 100 mL container with a stirrer chip and water to be 50 mL; and in a case where a time at which the water was added was set to 0 minutes, the mixture was stirred at 100 rpm and 1 mL of slurry was sampled 7 minutes later. The sampled slurry was immediately filtered through a 0.2 µm filter to obtain liquids BL1 and BL2, respectively. In a case where the cystine content of the liquid BL1 and the cystine content of the liquid BL2 were measured by high performance liquid chromatography (HPLC), the cystine content of BL1 was 0.15 g/L and the cystine content of BL2 was 0.09 g/L. The same procedure was carried out for B3, B4, B5, and B6 to obtain liquids BL3, BL4, BL5, and BL6, and the cystine contents thereof were 0.15 g/L, 0.09 g/L, 0.15 g/L, and 0.09 g/L, respectively. The pH of the slurry liquid before the sampling was acidic of 4 or more and less than 6.

In the present example, the initial cystine addition amount was at a concentration of 0.15 g/L, which is close to the solubility upper limit. However, in reality, it may not be possible to add up to the upper limit as the addition amount, and in a case of a powder in which the cystine and the specific component were added separately, since the cystine content detected after 7 minutes did not exceed 0.12 g/L even in consideration of the error, it can be said that the cystine mixed powder according to the embodiment of the present invention is considered to be contained in a case where the sampled filtration liquid after 7 minutes exceeded 0.12 g/L. The cystine concentration was preferably 0.12 g/L or more and 0.5 g/L or less, more preferably 0.13 g/L or more and 0.4 g/L or less, and most preferably 0.15 g/L or more and 0.3 g/L or less.

**[Table 5]**

| | Example 43 | Comparative Example 5 | Example 44 | Comparative Example 6 | Example 45 | Comparative Example 7 |
|---|---|---|---|---|---|---|
| Medium name | B1 | B2 | B3 | B4 | B5 | B6 |
| EMEM medium B (mg) | 595 | 595 | 595 | 595 | 595 | 595 |
| Mixed powder of present invention in which cystine/threonine = 1/4 (mass ratio) (mg) | 37.5 | 0 | 0 | 0 | 0 | 0 |
| Mixed powder of present invention in which cystine/serine = 1/4 (mass ratio) (mg) | 0 | 0 | 37.5 | 0 | 0 | 0 |
| Mixed powder of present invention in which cystine/tryptophan = 1/4 (mass ratio) (mg) | 0 | 0 | 0 | 0 | 37.5 | 0 |
| L-cystine (mg) | 0 | 7.5 | 0 | 7.5 | 0 | 7.5 |
| L-threonine (mg) | 0 | 30 | 0 | 0 | 0 | 0 |
| L-serine (mg) | 0 | 0 | 0 | 30 | 0 | 0 |
| L-tryptophan (mg) | 0 | 0 | 0 | 0 | 0 | 30 |

## Claims

1. A manufacturing method of a mixed powder, comprising:
a dissolving step of dissolving cystine and a specific component which is at least one selected from the group consisting of tryptophan, threonine, and serine in a solvent to obtain a solution containing the cystine and the specific component; and
a powdering step of drying the solution to obtain a mixed powder of the cystine and the specific component.

2. The manufacturing method of a mixed powder according to claim 1,
wherein, in the dissolving step, heating and/or an alkali is used for the dissolution.

3. The manufacturing method of a mixed powder according to claim 1,
wherein, in the dissolving step, heating is used for the dissolution.

4. The manufacturing method of a mixed powder according to claim 1,
wherein the powdering step is a step of drying the solution by spray drying and/or a step of drying the solution by heating and decompressing.

5. The manufacturing method of a mixed powder according to claim 1,
wherein, in the dissolving step, a mixed ratio of the specific component to the cystine is 1 to 10 in terms of mass ratio.

6. A mixed powder manufactured by the manufacturing method of a mixed powder according to claim 1.

7. A mixed powder comprising:
cystine; and
a specific component which is at least one selected from the group consisting of tryptophan, threonine, and serine,
wherein a solution obtained by dissolving the mixed powder in water at 15°C to 25°C for 7 minutes has a pH of 6.0 to 8.5, and
a cystine concentration of the obtained solution is 0.12 g/L or more.

8. A powder comprising:
cystine; and
a specific component which is at least one selected from the group consisting of tryptophan, threonine, and serine,
wherein, in a case where the powder is completely dissolved in water at 15°C to 25°C such that a final concentration of the cystine is 0.14 g/L or more, the following requirement is satisfied,
the requirement: in a case where a time when water is added with a water amount for adjusting the final concentration is set as 0 minutes, the solution is sampled after 7 minutes and filtered through a 0.2 µm filter, and the obtained liquid has a pH of 8.5 or less and a concentration of the cystine of 0.12 g/L or more.

9. A powder medium comprising:
the mixed powder or the powder according to any one of claims 6 to 8.
